# EUROPEAN PATENT APPLICATION

(11) **EP 1 398 041 A1**
(43) Date of publication of application: **17.03.2004**
(21) Application number: 02292255.3
(22) Date of filing: 13.09.2002
(51) Int. Cl.: A61K 48/00, C12N 15/867, A61P 27/02

(54) **Recombinant lentiviral vector pseudotyped with the hemagglutinin protein for gene transfer into the retina**

(71) Applicant: Université de Nantes, 44035 Nantes Cédex 01 (FR)
(72) Inventor: Rolling, Fabienne, 44000 Nantes (FR); Cosset, Francois-Loic, 69007 Lyon (FR)
(74) Representative: Desaix, Anne

(57) **Abstract**

The present invention relates to a novel lentiviral vector particularly well shaped for performing gene transfer into the retina. These lentiviral vectors are pseudotyped with a HA protein from an orthomyxovirus and comprise one or several gene(s) useful for preventing or treating diseases of the eye. The invention also relates to compositions and methods for preventing or treating diseases of the eye, using the vector of the invention to transfer selected genes suitable for preventing or treating diseases of the eye.

## Description

The present invention relates to a novel lentiviral vector particularly well shaped for performing gene transfer into the retina. The invention also relates to compositions and methods for preventing or treating diseases of the eye, using the vector of the invention to transfer selected genes suitable for preventing or treating diseases of the eye.

Retinal degenerative diseases such as retinal macular degeneration or *retinitis pigmentosa* constitute a broad group of diseases that all share one critical feature, the progressive loss of cells in the retina. There is currently no effective treatment available by which the course of these disorders can be modified and visual dysfunction eventually progresses to total blindness. Gene therapy represents a possible approach to treating retinal degenerations because the eye is easily accessible and allows local application of therapeutic vectors with reduced risk of systemic effects. Furthermore, transgene expression within the retina and effects of treatments may be monitored by a variety of non-invasive examinations.

Several viral vectors, including adenovirus, adenovirus-associated virus (AAV), or human immunodeficiency virus type 1 (HIV-1) were tested for *in vivo* retina transduction (see for example (Bainbridge, Stephens et al. 2001 )). Until now, an efficient long-term gene transfer was documented for the AAV2-based vectors only. These vectors are able to transduce both the photoreceptors and the retinal pigment epithelium (RPE) after subretinal administration. However, the transgene expression is delayed. Maximal levels of expression are detected 4-6 weeks post-infection (Rolling, Shen et al. 1999; Auricchio, Kobinger et al. 2001). Such a feature may be detrimental when rapid transgene expression is required.

Gene transfer into the retina was recently reported using HIV vectors pseudotyped with vesicular stomatitis virus G glycoprotein (VSV-G), but the tropism remains unclear. Subretinal injection in rats and mice leads to the transduction of both photoreceptor cells and RPE (Miyoshi, Takahashi et al. 1997; Auricchio, Kobinger et al. 2001). In contrast, Brainbridge et al. demonstrated that the transduction is restricted to the RPE in mice (Bainbridge, Stephens et al. 2001).

The inventors have now developed a novel lentiviral vector which exhibits a very low toxicity and high efficiency, for performing gene transfer into the eye. The present invention hence provides compositions and methods for preventing and treating a number of diseases of the eye, including for example, retinal macular degeneration or *retinitis pigmentosa,* as well as other diseases such as neovascular diseases. The present invention also provides other related advantages.

The inventors have developed a lentiviral vector derived from SIVmac251, a nonpathogenic strain of simian immunodeficiency virus (SIV). It was previously demonstrated that HIV-1 and SIV vectors could transduce both dividing and resting cells in vitro with equivalent efficiencies (Mangeot, Negre et al. 2000; Negre, Mangeot et al. 2000). To assess both, the virus tropism in retina and the kinetic of the transgene expression, rats were injected with VSV-G-pseudotyped SIV vectors encoding for the green fluorescent protein (GFP). Sustained gene transfer into the retina was monitored by iterative non-invasive examinations (examples 1 and 2). Since the tropism is mainly determined by the glycoproteins expressed onto the virions, the inventors evaluated the cell populations transduced by SIV vectors pseudotyped with surface glycoproteins from other membraneenveloped viruses and, particularly, with the hemagglutinin (HA) protein from an orthomyxovirus, more precisely from an influenza A virus. They demonstrated that the HA-pseudotyped vectors were very efficient (example 5).

The present invention hence mainly pertains to lentiviral vectors pseudotyped with a HA protein from an orthomyxovirus and comprising one or several gene(s) useful for preventing or treating diseases of the eye, pharmaceutical compositions comprising such vectors, and uses of these vectors.

Throughout this application, several words are employed, the meaning of which should be understood according to the following definitions :
In this application, the word "Orthomyxovirus" refers to any wild-type virus of the Orthomyxoviridae family, as defined in *Virology*, Ed. B. N. Fields, New York, Raven Press, chapters 45 and 46. In the present application, "Orthomyxovirus" also refers to any natural mutant of said viruses, as well as to any recombinant virus derived from said viruses. Particular orthomyxoviridae are Influenza A, B and C viruses, which all have spikes projecting radially outward the surface of the virions. On influenza A, these spikes are of three distinct types, corresponding to the hemagglutinin protein (HA), the neuraminidase (NA), and the M2 component of the virus. The latter component is present in small number and is not found in influenza B strains. Influenza C viruses possess only one surface spike with both HA and esterase activity (see, for example, *Virology*, supra, p. 1400, column 1, last paragraph of the third edition).

In the present application, the term "HA" will hence designate the hemagglutinin protein of any kind of orthomyxovirus, in particular of any influenza virus including the surface glycoprotein of influenza C and, more particularly, the hemagglutinin of an influenza A virus. Influenza A viruses naturally infect a broad range of species, including humans, horses, swine, and a wide variety of avian species. Their surface glycoproteins, including HA, exhibit a great amino acid sequence variability. Therefore, the word "HA" in the present application designates a wide range of proteins that are
characterized by their origin (orthomyxoviridae), and their function (hemagglutination).

Usually, the phrase "viral vector" designates either a viral particle comprising genetic material to be transferred into a host cell, or the recombinant viral genome itself. Throughout this specification, and for clarification purpose, the term "(lenti)viral vector particle" will be used to designate the physical viral particle, including a genome, a capsid and an enveloppe, whereas "(lenti)viral vector genome" will designate the nucleic acid construct to be transferred. When neither one nor the other term is specified, both can be understood, depending on the context.

Throughout this application, when two elements are said "operably linked", this means that they are configured so as to interact in their usual way, to perform their usual function. For example, when a coding sequence is operably linked to a promoter or any other transcription control element, this means that this coding sequence will be transcribed under the control of this promoter or these elements.

The present invention hence provides a lentiviral vector particle comprising a lentiviral vector genome encoding a transgene which, when expressed in the retina, can prevent, inhibit or alleviate the effects of an eye disease, wherein said vector particle is pseudotyped with an hemagglutinin (HA) protein of an orthomyxovirus. A particular lentiviral vector particle of the invention is pseudotyped with an HA protein from an influenza A virus, and, for example, from a Fowl Plague Virus (FPV). The results shown in the following experimental part have been obtained with lentiviral particles pseudotyped with an HA protein from strain *influenza* A, Fowl plague virus, Rostock, 34 (H7 N1). The sequence of this HA protein is accessible in GenBank under numbers M24457 (coding sequence) and AAA43150 (amino acids sequence).

As described in the experimental part, HA-pseudotyped vectors can be produced by co-transfecting plasmids, wherein one plasmid carries the vector genome and the others encode the necessary proteins for lentiviral encapsidation and the HA protein. The inventors have demonstrated that cotransfection of a further plasmid, encoding the neuraminidase (NA) from FPV, could result in improved vector production, with increased titers (titers of 10⁸ infectious particles/ml were obtained after a 100-fold concentration of the collected supernatant, as described below). The present invention hence also pertains to a lentiviral vector particle comprising a lentiviral vector genome encoding a transgene which, when expressed in the retina, can prevent, inhibit or alleviate the effects of an eye disease, wherein said vector particle is pseudotyped with both an hemagglutinin (HA) and a neuraminidase (NA) proteins of an orthomyxovirus, preferably from an *influenza* virus, and more preferably from *influenza A.* One particular NA protein which can be comprised in the vector particles according to the invention is the NA protein from FPV, for example from the strain Rostock, 34 (H7 N1). The sequence of this NA protein is accessible in GenBank under numbers X52226 (coding sequence) and CAA36475 (amino acids sequence).

The invention also pertains to lentiviral vector particles such as described above, wherein said vector genome comprises a lentiviral 5' LTR, a tRNA binding site, a packaging signal, a promoter operably linked to said transgene, an origin of second strand DNA synthesis and a 3' lentiviral LTR.

According to a particular embodiment of the invention, the vector genome within the lentiviral vector particle comprises 5' and/or 3' LTRs from a virus selected from the group consisting of SIV, HIV, HIV-1, HIV-2, EIAV and FIV.

According to another particular embodiment of the present invention, the lentiviral vector is self-inactivating. Self-inactivating vectors can be made by deleting promoter and enhancer elements in the U3 region of the 3' LTR, including the TATA box and binding sites for one or more transcription factors. The deletion is transferred to the 5' LTR after reverse transcription and integration in transduced cells. This results in the transcriptional inactivation of the LTR provirus. Such self-inactivating (SIN) vectors can present the advantage of a broader tropism. Indeed, Miyoshi *et al.* have suggested that the HIV-1 LTR can negatively influence the transgene expression through an internal promoter in some cell types, and hence that SIN vectors could present a double advantage in terms of safety and broader applicability, particularly concerning the photoreceptor cells (Miyoshi, Blomer et al. 1998).

Utilizing the methods and gene delivery vectors provided herein a wide variety of diseases of the eye may be readily treated or prevented, including for example, glaucoma, macular degeneration, diabetic retinopathies, inherited retinal degeneration such as *retinitis pigmentosa,* retinal detachment or injury and retinopathies (whether inherited, induced by surgery, trauma, a toxic compound or agent, or, photically). Similarly, a wide variety of neurotrophic factors may be utilized (either alone or in combination) within the context of the present invention, including for example, NGF, BDNF, CNTF, NT-3, NT-4, FGF-2, FGF-5, FGF-18, FGF-20 and FGF-21.

Within certain embodiments of the invention, it is preferred that the gene delivery vector be utilized to deliver and express an anti-angiogenic factor for the treatment, prevention, or, inhibition of diabetic retinopathy, wet ARMD, and other neovascular diseases of the eye (e.g., ROP). Within other embodiments it is desirable that the gene delivery vector be utilized to deliver and express a neurotrophic growth factor to treat, prevent, or inhibit diseases of the eye, such as, for example, glaucoma, *retinitis pigmentosa,* and dry ARMD.

Within further aspects of the present invention gene delivery vectors are provided which direct the expression of a neurotrophic factor, or, an anti-angiogenic factor. As noted above, representative examples of neurotrophic factors include NGF, BDNF, CNTF, NT-3, NT-4, FGF-2, FGF-5, FGF-18, FGF-20 and FGF-21. Representative examples of anti-angiogenic factors include soluble Flt-1, soluble Tie-2 receptor, and PEDF.

In the lentiviral vector particle according to the invention, the transgene can hence be selected (without being limitative) in the group comprising neurotrophic factors including NGF, BDNF, CNTF, NT-3, NT-4, FGF-2, FGF-5, FGF-18, FGF-20 and FGF-21, anti-angiogenic factors including soluble Flt-1, soluble Tie-2 receptor, and PEDF, and enzymes including β-glucuronidase, neuraminidase, sphingomyelinase, sulfatases, arylsulfatase β, α-neuraminidase, gangliosidase, tripeptidyl protease, CLN3 and palmitoyl protein thioesterase (PPT).

In these lentiviral vectors, the transgene is preferably operably linked to a regulated promoter (*e.g.*, "tet" promoters, the ecdysome system, or other systems of regulation), a viral promoter (*e.g.*, the CMV or RSV promoters), a tissue specific promoter (*e.g.*, a rod, cone, or ganglia-derived promoter), or a rhodopsin promoter.

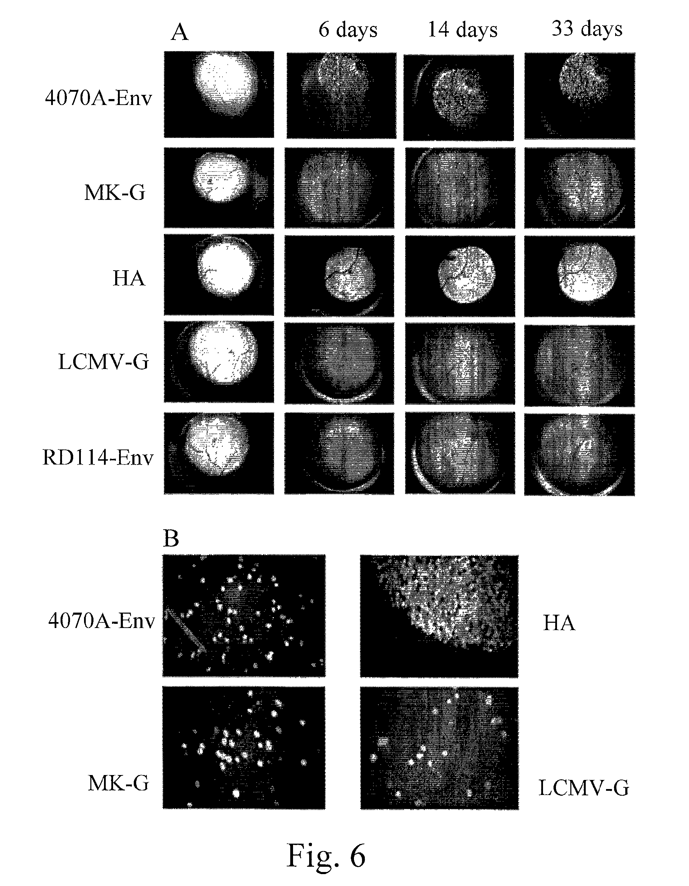

The present invention also pertains to a pharmaceutical composition for administration in the eye, comprising lentiviral vector particles according to the invention (as described above), and a pharmaceutically acceptable carrier. A particular advantage of the vectors according to the invention is that time-course transductions showed that high levels of transgene expression were obtained as soon as 4 days post-injection. For the first time, the kinetic of the transgene expression could be monitored *in vivo* in lentivirally-infected cells by iterative non-invasive examinations (see examples 1 and 2). Moreover, the HA-pseudotyped vector showed an excellent transduction efficiency (see, Fig. 6A), and lower toxicity (Example 6). This early onset of a robust expression of the transgene, and other advantages of the vector of the invention, are attractive features for degenerative retinal diseases showing rapid photoreceptors degeneration. A recent study demonstrated correction of the retinal dystrophy phenotype of the RCS rat by adenoviral gene transfer of Mertk. The *rdy* defect resides in the RPE, and rescue needs immediate and strong RPE therapeutic gene expression that matches the rapid degeneration in RCS rat. In this model, HA pseudotyped lentivectors would represent the optimal choice.

Another aspect of the invention is the use of a lentiviral vector particle as described above, for the manufacture of a composition for treating a disease in the eye or preventing cell damage in the eye. In a particular embodiment of this aspect of the invention, the disease is due to a deficiency in retinal pigment epithelium (RPE) cells.

Alternatively, the disease can be due to a deficiency in the photoreceptors. In this latter case, lentiviral particles which are able to transduce retinal pigment epithelium (RPE) cells and express a neurotrophic factor therein can be used according to the invention. The diffusion of the neurotrophic factor around the transduced cells will attain the photoreceptors and have a beneficial effect on them.

According to the invention, and depending on the transgene introduced into the vector, the lentiviral vector particles described above can be used for the manufacture of a composition for treating a neovascular disease of the eye, a glaucoma, a macular degeneration, a diabetic retinopathy, an inherited retinal degeneration, a retinitis pigmentosa, a cell damage in retinal epithelial cells associated with Sly syndrome, a retinal detachment or injury or a retinopathy.

A further aspect of the present invention is the use of an hemagglutinin (HA) protein of an orthomyxovirus, to pseudotype an enveloped vector so that said vector is able to transduce efficiently retinal pigment epithelium (RPE) cells. In a particular embodiment of this aspect of the invention, the HA protein is from an influenza A virus and, for example, from a Fowl Plague Virus. More particularly, the HA protein can be from the strain Rostock 34 (H7 N1) mentioned above.

The present application also relates to a method of treating, inhibiting or preventing a disease of the eye, comprising, administering intraocularly, in a mammal in need thereof, a gene delivery lentiviral vector particle which is pseudotyped with an orthomyxovirus hemagglutinin (HA) protein and which directs the expression of a transgene wherein said transgene, when expressed in the retina, can prevent, inhibit or alleviate the effects of an eye disease. As already mentioned above, the HA protein can be from an influenza A virus, *e.g.*, a Fowl Plague Virus, *e.g.*, strain *influenza A*, FPV, Rostock, 34 (H7 N1). The sequence of this HA protein is accessible in GenBank under numbers M24457 (coding sequence) and AAA43150 (amino acids sequence).

The above method can be performed according to the invention, when the disease is due to a deficiency in retinal pigment epithelium (RPE) cells, or when the disease is due to a deficiency in the photoreceptors. In this latter case, the method can be performed with lentiviral particles that transduce retinal pigment epithelium (RPE) cells and express therein a neurotrophic factor, which will diffuse to the photoreceptors.

These methods can be performed to treat, inhibit or alleviate a variety of diseases of the eye, including, without being restrictive, a neovascular disease of the eye, a glaucoma, a macular degeneration, a diabetic retinopathy, an inherited retinal degeneration, a *retinitis pigmentosa,* a cell damage in retinal epithelial cells associated with Sly syndrome, a retinal detachment or injury or a retinopathy.

Within yet other embodiments, it may be desirable to utilize either a gene delivery vector which expresses both an anti-angiogenic molecule and a neurotrophic growth factor, or two separate vectors which independently express such factors, in the treatment, prevention, or inhibition of an eye disease (e.g., for diabetic retinopathy).

Within further embodiments of the invention, the above-mentioned methods utilizing gene delivery vectors may be administered along with other methods or therapeutic regimens, including for example, photodynamic therapy (e.g., for wet ARMD), laser photocoagulation (e.g., for diabetic retinopathy and wet ARMD), and intraocular pressure reducing drugs (e.g., for glaucoma).

While the invention has been described in terms of various preferred embodiments, the skilled artisan will appreciate that various modifications, substitutions, omissions and changes may be made without departing from the scope thereof. Accordingly, it is intended that the scope of the present invention be limited by the scope of the following claims, including equivalents thereof.

Other characteristics of the invention will also become apparent in the course of the description which follows of the biological assays which have been performed in the framework of the invention and which provide it with the required experimental support, without limiting its scope.

### BRIEF DESCRIPTION OF THE DRAWINGS

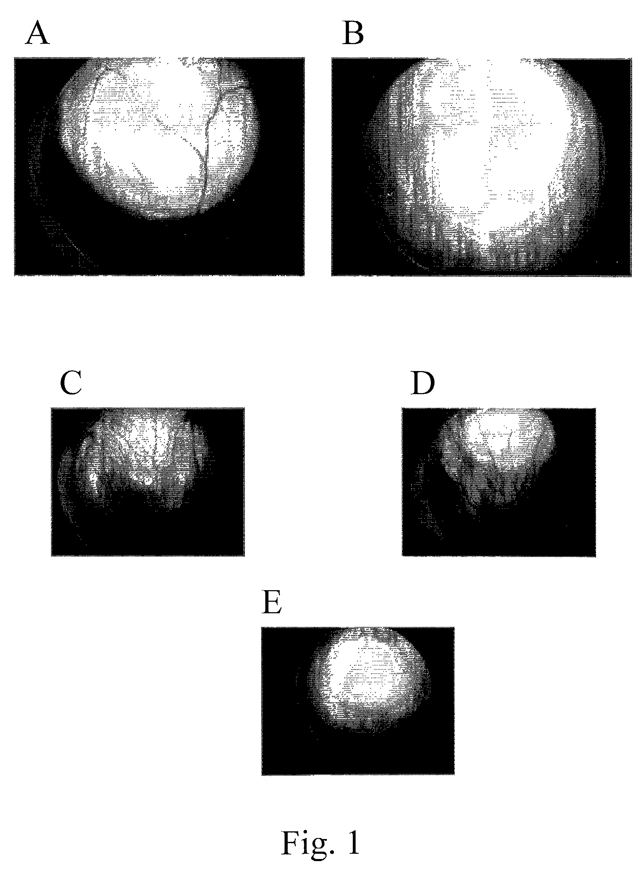

**FIG.1** Assessment of vector administration : diluted fluorescein was added to the viral solution, and accuracy of the injection was monitored by fluorescence fundus photography. **A:** subretinal injection **B:** intravitreal injection **C:** subchoroïdal injection **D:** mixed subretinal and subchoroïdal injection **E:** mixed subretinal and intravitreal.

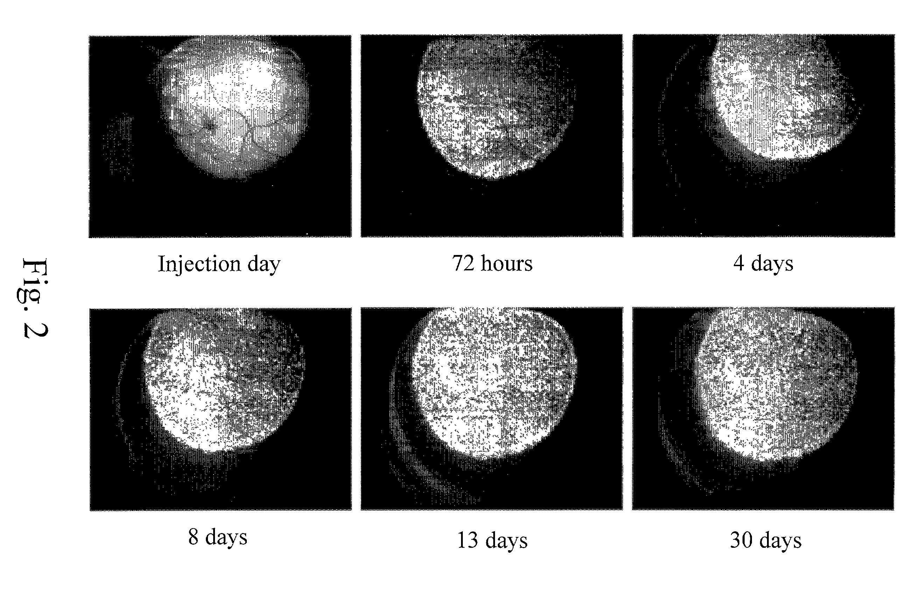

**FIG.2** Retinal imaging showing GFP expression at different time points after VSV-G pseudotyped SIVgfp subretinal injection. Efficient transgene expression was detected as early as 72 hrs post injection, was maximal at 4 days and remained stable thereafter. As shown, each bright spot on the retinal images correspond to a single transduced RPE cell (typical hexagonal shape of the RPE cells).

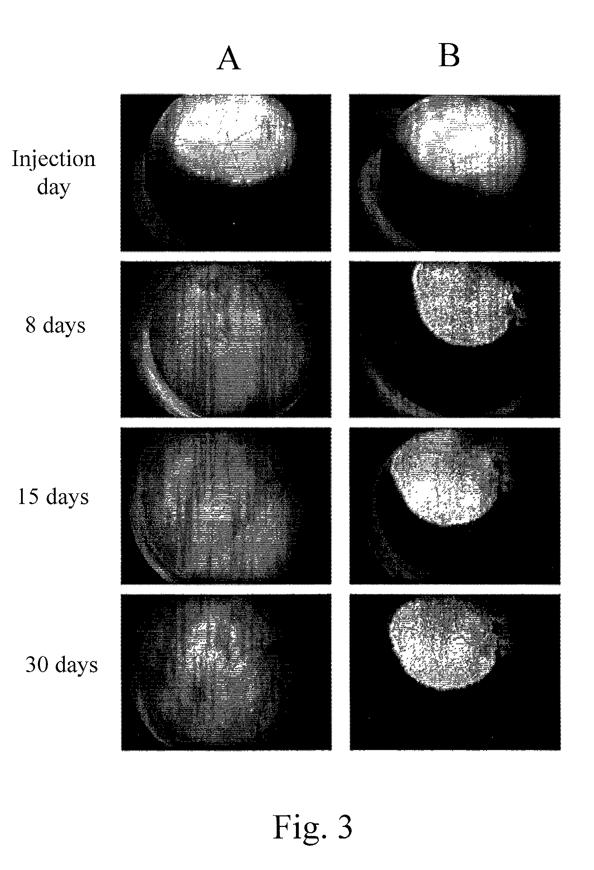

**FIG.3** Evaluation of pseudotransduction: recombinant pseudoSIV.*gfp* (VSV-G pseudotyped SIV empty particles) **(A)** and SIV.*gfp* **(B)** were silmutaneously injected in the subretinal space in the right and left eyes of the same animal, respectively. No GFP signal was ever detected with pseudoSIV.*gfp*, while a normal pattern of transgene expression was detected with SIV.*gfp* vector. This result indicated that early onset of transgene expression was due to active gene transfer rather than pseudotransduction.

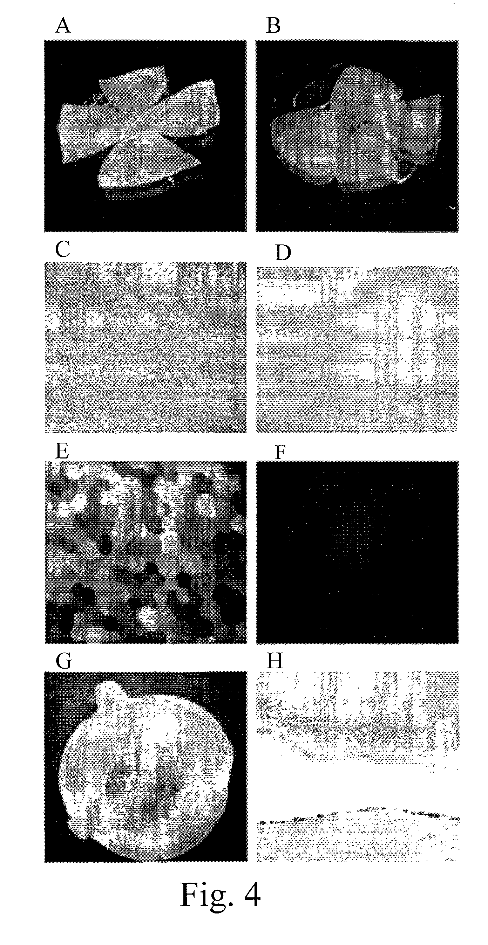

**FIG.4** VSV-G pseudotyped SIV.*gfp* and SIV.*lacZ* tropism following subretinal injection: Retina flamounts were performed on SIV.*gfp* injected eyes by separating the sclera/choroid/RPE layers **(A)** from the neuroretina **(B)** and observed under inverted fluorescence microscopy. The sclera/choroid/RPE **(C)** displayed GFP signal within the RPE cells **(E).** Original magnification: x20. No GFP signal was detected in the neuroretina **(D,F)**. Original magnification: x20. This result indicated that no photoreceptor or other neuronal cells were transduced.
   SIV.*lacZ* injected eyes were enucleated, eye cups were fixed and stained by X-gal **(G).** After histological sections, X-Gal staining was observed exclusively in the RPE cell layer **(H)**. Original magnification: x20. These results demonstrated that accurate subretinal injection of pseudotyped SIV.*gfp* allowed specific transduction of the RPE cells.

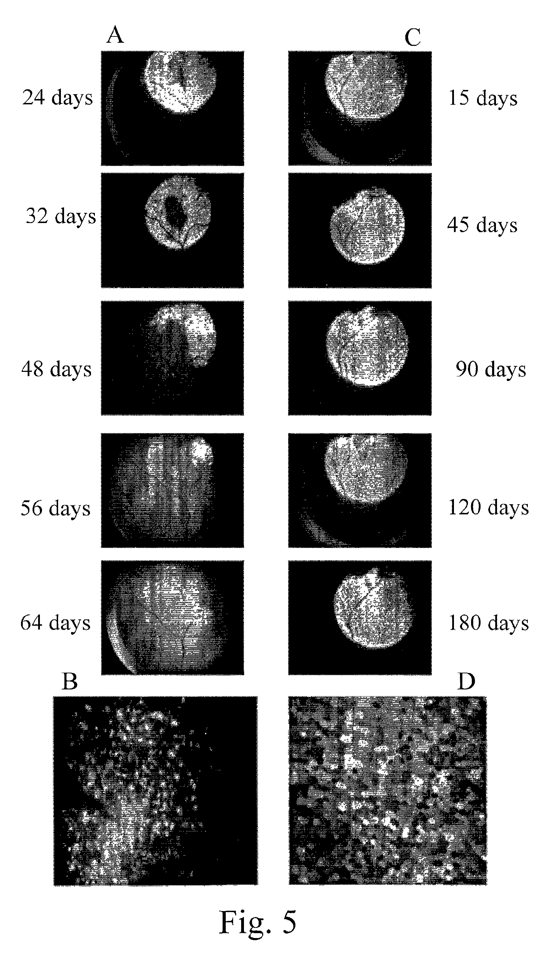

**FIG.5** Long-term expression and GFP protein toxicity: While VSV-G pseudotyped SIV-mediated gene transfer remained stable for ~one month, progressive loss of GFP signal was observed, thereafter **(A)**. RPE damage restricted to the transduced area could be observed by inverted fluorescence microscopy on the sclera/choroïd/RPE flatmount (B). Subretinal injection of VSV-G pseudotyped SIV carrying a low toxicity *gfp* cDNA showed long-term expression and preserved RPE architecture **(C).** Additionally, sclera/choroïd/RPE displayed normal transduced RPE morphology six month post-injection **(D).**
**FIG.6** Evaluation of gene transfer efficiency and tropism of 4070A-Env, MK-G, HA, LCMV-G and RD114-Env pseudotyped SIV.*gfp* following subretinal injection. Retinal imaging showing GFP expression at different time points **(A)**. No GFP signal could be detected with RD114-Env pseudotyped SIV.*gfp.* However, 4070A-Env, MK-G- and HA-pseudotyped SIV displayed the same kinetic of expression with HA showing the most efficient transduction. LCMV-G displayed a delayed onset of expression and reached a plateau three weeks post-injection. For 4070A-Env, MK-G-, HA- and LCMV-G-vectors, GFP signal was detected within the RPE cells on the sclera/choroid/RPE layer **(B)**. No signal was ever detected in the neuroretina layer (not shown).

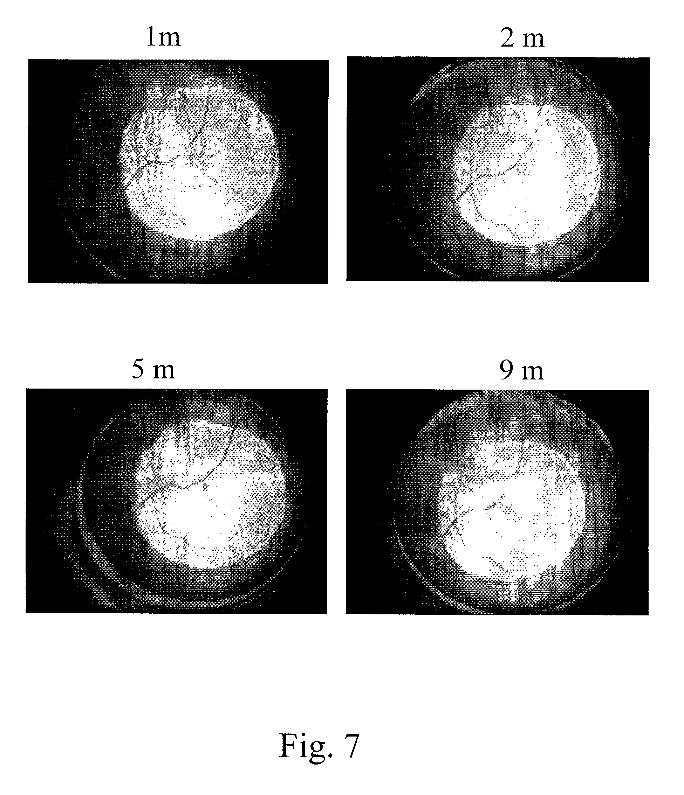

**FIG.7** Long-term expression 1, 2, 5 and 9 months after subretinal injection of HA-pseudotyped SIV carrying *gfp* cDNA : the follow-up observation of the animal injected with the HA-vector as shown in Figure 6 show that the level of transgene expression is maintained over at least 9 months.

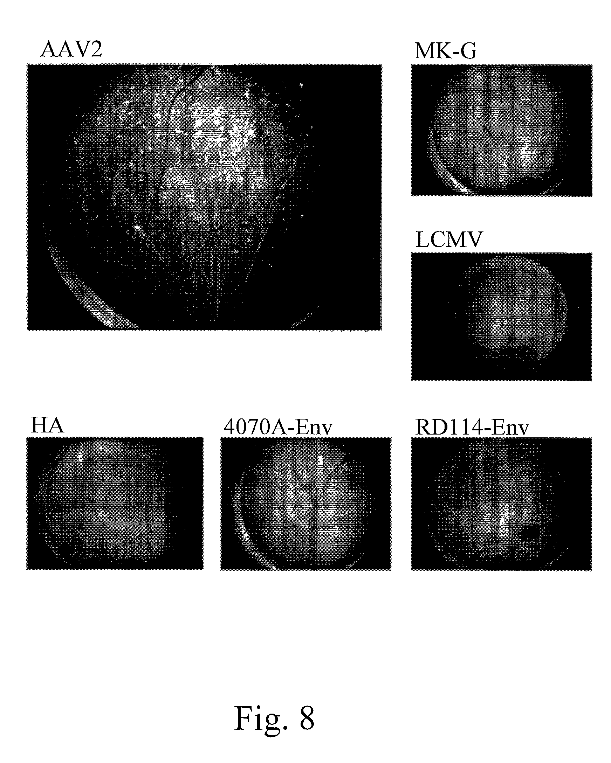

**FIG.8** Evaluation of gene transfer efficiency of 4070A-Env, MK-G-, HA-, LCMV-G- and RD114-Env pseudotyped SIV.*gfp* following intravitreal injection. No GFP signal could be detected on the fluorescence fundus photography at 6, 14 and 21 days post-injection. Intravitreal injection of rAAV2.gfp was used as a positive control and displayed transduced ganlion cells with optical fiber converging to the optical nerve head.

### EXAMPLES

The following examples can be performed using the materials and methods described below:

### Materials and Methods

### Cells and plasmids.

293 embryo kidney cell line (ATCC CRL-1573) was grown in Dulbecco's modified Eagle's medium (DMEM; Sigma) supplemented with 10% fetal calf serum (FCS).

SIV packaging constructs were derived from the BK28 molecular clone of SIVmac251 (GenBank accession number M19499). pSIV3 (encoding for Gag-Pol, Tat, Rev, Vif, Vpx, Vpr) and pSIV4 (encoding for Gag-Pol, Tat and Rev) were previously described in (Negre, Mangeot et al. 2000). pRMES4SA harboring the recombinant SIV.*gfp* vector genome derived from the pRMES4 described in (Negre, Mangeot et al. 2000). Briefly, the vector genome contains the LTRs, the packaging signal overlapping the 57 first nucleotides of the GAG gene, the cPPT/CTS region (nt 5049-5189), the extended RRE sequence (nt 8057-8808), an internal CMV promoter and the gene encoding the enhanced GFP protein (eGFP, Clontech). For the construction of the SIV.*ltgfp* vector, the hr*gfp* gene was extracted from the pIRES-hr*gfp* plasmid (Stratagene) by BSTXI-Kpnl, blunt-ended by Klenow fragment digestion and ligated to *Bam*HI linkers. The 700-bp fragment was finally inserted into the *Bam*Hl sites within the pRMES4SA plasmid, in place of the e*gfp* gene. For the construction of the SIV.*lacZ* vector, LacZ gene was extracted from the pMFG-LacZ plasmid (Duisit, Salvetti et al. 1999) and inserted into the pRMES4SA BamHl sites. Finally, plasmids were used to express the envelope glycoproteins. phCMV-G (Negre, Mangeot et al. 2000), and phCMV-4070A (Sandrin, Boson et al. 2002) were previously described. phCMV-HA (Hatziioannou, Valsesia-Wittmann et al. 1998) was obtained by deleting the VSV-G coding sequence from the phCMV-G plasmid [Yee, 1994 #12] and inserting in place thereof the *Bg*/II/*Bg*/II fragment comprising the coding sequence of the FPV hemaggluntinin (GenBank accession number M24457), upstream from the rabbit betaglobin intron II sequence. The phCMV-NA plasmid was obtained by inserting the cDNA comprising the coding sequence of FPV neuraminidase (GenBank accession number X52226) into the EcoRI site of pCMV-G after excision of the gene encoding VSV-G. In both phCMV-HA and phCMV-NA, the glycoprotein gene (HA or NA) is under the control of the CMV immediate early promoter. phCMV-RD114 was further modified to express the RD114/TR chimeric glycoprotein carrying the 4070-ENV cytoplasmic tail (Sandrin, Boson et al. 2002). pMK-G was a gift from JM Heard, Pasteur (Desmaris, Bosch et al. 2001).

The phCMV-NA plasmid encodes the FVP neuraminidase, the sequence of which is available at the GenBank under number X52226. This sequence is under the control of the CMV i.e. promoter.

### Recombinant SIV production.

Production of recombinant vectors was achieved by three-plasmid transfection into 293 cells, using pSIV3 or pSIV4, the plasmids coding for the respective envelopes and for the vector genomes.

Production experiments showed that lentiviral vectors generated with the FPV hemagglutinin could not egress from producer cells after GP assembly. Indeed, when vector-producer cells expressing the FPV-HA were treated with purified neuraminidase, infectivity of HA-pseudotyped vectors was strongly increased by up to 100-fold. This enhancement correlated with a 50-fold increased production of viral particles in the supernatant of producer cells. This was induced by neuraminidase-mediated release of virions from the cell surface on which they were retained because of binding to sialic acid-containing cell surface molecules. Therefore, a fourth plasmid, encoding the FPV neuraminidase protein, was also co-transfected for HA-pseudotyped vectors. The phCMV-NA plasmid was used therefor.

Large-scale productions were generated according to the protocol described by Patrick Salmon at the Trono's laboratory web site (http://www.medecine.unige.ch/~salmon/) and by Negre et al. (2000). Briefly, 293T cells were seeded in 10 cm culture plates (2.4 x 10⁶ cells/plate) and co-transfected the next day by calcium phosphate precipitation. For producing the HA-pseudotyped vectors, 8.1 µg of plasmid encoding the lentiviral capsid, 8.1 µg of plasmid comprising the vector, 2.7 µg of HA-coding plasmid, and 2.7 µg of phCMV-NA were co-transfected. The culture medium (DMEM + 101% FCS; 12 ml/plate) was changed 16 hrs later. Cell culture supernatants were recovered 2 and 3 days post-transfection, filtered through 0.45 µm pore-sized membranes, and ultracentrifuged. Pellets were carefully resuspended in Dulbecco's Phosphate Buffered Saline supplemented with calcium and magnesium (BioWhittaker Europe, Belgium). For the production of pseudoSIV.*gfp* particules, pGFP-NI (Clontech) plasmid was transfected in place of the pRMES4SA plasmid.

### Vectors titration.

For the titration of the GFP-encoding vectors, 293 cells were plated at 2.5x10⁵ cells/well in 6-well plates 1 day before transduction. Infectious supernatants were serially diluted in DMEM plus 10% FCS, 8 µg/ml polybrene. Target cells were maintained in culture for 3 days. Percentages of GFP-positive cells were determined by FACS analysis as described in (Negre, Mangeot et al. 2000). For the titration of the SIV.lacZ vectors, 293 cells were plated at 1x10⁵ cells/well in 24-well plates 1 day before transduction. Infectious supernatants were serially diluted in DMEM plus 10% FCS, 8 µg/ml polybrene. Target cells were maintained in culture for 2 days before X-gal staining. For the quantitative determination of reverse-transcriptase contents, serially-diluted aliquots of stocks were used for the determination of the reverse-transcriptase activity using a non-radioactive RT assay (Roche).

### Subretinal and intravitreal injections

Rats used for this study were cared for in accordance with the ARVO statement for the use of animals in ophthalmic and vision research. Wistar rats were anaesthetized with intramuscular injection of Ketamine/Xylazine (50 mg/kg and 6mg/kg respectively).

Subretinal injections were performed via a transscleral transchoroidal approach on wild-type Wistar rats as previously described (Rolling, Shen et al. 1999). Briefly, the sclera and the choroïd were punctured; a 33-gauge needle was then inserted in a tangential direction under an operating microscope. Fluorescein was extemporarily added to the viral solution at a 1/1000 final dilution and the injection mixture was delivered into the subretinal space.

For intravitreal injections, the needle tip was advanced through the sclera posterior to the corneoscleral limbus into the vitrous cavity. Accuracy of subretinal and intravitreal injections were monitored by fluorescence fundus photography immediately following the injection procedure.

### In vivo GFP fluorescence imaging

GFP protein expression in live rats was monitored by fluorescence retinal imaging by using a Canon UVI retinal camera connected to a digital imaging system (Lhedioph win software). Retinas were examined at dayly and weekly intervals following injection. Identical experimental conditions and parameters were used for fluorescence fundus photography at each time point.

### RPE-choroid-sclera and neuroretina flatmounting

The enucleated eyes were fixed with 4% paraformaldehyde for 1 hr at room temperature. After washing in PBS, the eyes were cut through the pars plana, the anterior segment and the lens were removed. The eye cup was cut peripherally in four sections under an operating microscope and flattened. The neuroretina was removed from the RPE-choroid-sclera with fine forceps, both cell layers were mounted on a glass slide and were examined by fluorescence microscopy.

### Tissue sections

Fixed neuroretina flatmount was embedded in O.C.T. compound (Sakara Finetek, Torrance, CA) and snap frozen in liquid nitrogen. Cryosections (10-12 µm) were cut and observed by fluorescence microscopy. For paraffin sections, eyes were fixed in 4% paraformaldehyde for 24 hrs, removed and the eye cups stained in 5-bromo-chloro-3-indolyl-β-D-galactopyranoside (X-Gal) overmight. The eyes were then embedded in paraffin and sectioned.

### Example 1 : Accurate assessment of vector administration

The small size of rodent eyes makes precise intravitreal, and more evidently, subretinal injection difficult to control. In order to assess vector administration, diluted fluorescein was added to the viral solution, and accuracy of the injection site was monitored by fluorescence fundus photography immediately after vector delivery. Therefore, detection of retinal vessels over the surface of the green bleb characterized a restricted subretinal administration (Fig. 1A), whereas retinal vascularization is completely occulted by the dye which instantly diffuses with fusy border (Fig1B) as a result of intravitreal injection. As observed in Fig. 1C, 1D and 1E, subchoroïdal (choroidal vascularization) and mixed injections could occur (subretinal/subchoroïdal, subretinal/intravitreal), and, without the fluorescein marker, could not be distinguished solely by direct vision using the operating microscope. Dye diffusion was followed by fluorescence fundus photography at various time points post subretinal and intravitreal administration, and no fluorescein could be detected 24 hrs post-vector injection.

To compare gene transfer efficiencies and SIV pseudotypes tropism, only animals that have received a true and restricted subretinal or intravitreal injection were included in the study.

### Example 2 : Efficient gene transfer in the retina with VSV-G pseudotyped SIV following subretinal injection

Subretinal injection of VSV-G pseudotyped SIV.*gfp* (10⁸ transducing units (TU)/ml corresponding to 2 10⁵ tu/injection) were performed on six Wistar rats. For each animal, retinal images showed efficient transgene expression within the detached portion of the retina as early as 72 hrs post injection. At four days post injection, GFP expression was maximal and remained stable for more than one month (Fig. 2).

It was previously demonstrated that ultracentrifugation of VSV-G-pseudotyped MLV vectors encoding for the ß-Gal leads to the copurification of the enzyme. Consequently, injection of concentrated virus stocks may result in high rates of protein transfer rather than gene transfer, a process referred to as *pseudotransduction.* To ensure the fluorescent signal detected four days post-injection was actually due to newly synthesized GFP, a stock of VSV-G-pseudotyped SIV empty particles was produced on 293 cells, except that the pRMES4SA plasmid containing the vector genome was replaced by an unrelated plasmid harboring the *gfp* expression unit alone but no encapsidation signal. In this setting, GFP cDNA cannot be encapsidated within the particles, and only the GFP protein can eventually associate with the virions or be packaged in copurifying lipid vesicles. This preparation, called pseudoSIV.*gfp*, was concentrated by ultracentrifugation and further processed as a standard SIV vector production (see Material & Methods). PseudoSIV.*gfp* (right eye) and SIV.*gfp* (left eye) vectors were subretinally injected in parallel in 3 rats. In none of these animals, GFP detection occured in the pseudoSIV.*gfp* injected eye (Fig. 3A) even at 24 to 48 hrs post administration, while intense fluorescence was consistently detected in the SIV.*gfp* injected eye (Fig. 3B). This result confirmed that the early onset of GFP detection is due to active gene transfer.

### Example 3 : VSV-G-pseudotyped SIV tropism is restricted to the RPE.

Fluorescence fundus photography of subretinally injected eyes strongly suggested that RPE cells were the actual transduced cells (Fig. 2 and Fig. 3). To further characterize the transduced cells within the retina, animals were sacrificed and the SIV.*gfp*-injected eyes were enucleated. Retina flatmounts were performed and the sclera/choroid/RPE layer was separated from the neuroretina (Fig. 4A and B). By examination under a fluorescence inverted microscope, GFP signal was only observed on the sclera/choroid/RPE layer and showed that RPE cells were efficiently transduced (Fig. 4E). No signal was detected in the neuroretina layer (Fig. 4F). To confirm the flatmount results, four animals were injected subretinally with VSV-G-pseudotyped SIV.*lacZ* vectors. Seven days post-injection, animals were sacrificed and histological sections were performed. X-Gal staining was observed only in the RPE cell layer (Fig. 4H).

### Example 4 : Stable SIV-mediated RPE transduction persists at least 6-month (duration of the experiment).

While VSV-G-pseudotyped SIV.*gfp*-mediated gene transfer remained stable for more than one month, loss of GFP signal was seen, thereafter (Fig. 5A). Concomitantly, RPE damage restricted to the transduced area was documented by inverted fluorescence microscopy on the sclera/choroïd/RPE flatmount (Fig. 5B). As monitored on the six injected eyes, the rapidity and severity of RPE destruction correlated with the amount of GFP expression.

No toxicity related to the use of lentivirus vectors was reported yet. However, the cytotoxicity of the GFP protein itself was previously mentionned (Doi, Hargitai et al. 2002). Therefore, a new recombinant vector was generated, encoding the low-toxicity GFP (from mutant *Renilla reniformis)* instead of the enhanced GFP (from *Aequorea victoria*) used initially. Recombinant SIV.*gfp* and SIV.*Itgfp* vectors were produced at equivalent titer (10⁸ TU/ml) and were subretinally injected in 6 Wistar rats. Fig. 5C shows that no rejection phenomenon occurred in any of these animals receiving the SIV.*Itgfp* vector and long-term GFP expression remained stable over a six-month period. Additionally, sclera/choroïd/RPE flamount showed normal RPE morphology of transduced RPE cells six months post-injection (Fig. 5D).

### Example 5 : Comparative evaluation of five SIV.gfp pseudotypes.

The inventors demonstrated a sustained GFP expression in RPE following subretinal injection of a VSV-G pseudotyped SIV.*gfp* vector. To exploit the powerful potential of envelope substitution on lentiviral vectors, other pseudotypes were compared, including MK-G, 4070A-Env, HA, LCMV-G and RD114-Env glycoproteins. Titers determined by end-point dilutions on 293 cells, showed differences among the various pseudotypes. Vectors pseudotyped with RD114-Env and HA were found at 10⁸ TU/ml, while MK-G, 4070A-Env and LCMV-G pseudotypes were 100-fold less infectious. This discrepancy could reflect variable expression of the related receptors onto the 293 cells rather than a misassembly of the particles. Therefore, productions were normalized according to the reverse-transcriptase (RT) activity measured in the concentrated stocks. Equivalent amount of total particles, corresponding to an average of 5 ng of RT, were injected in the following experiment.

Subretinal injections of each pseudotype were performed in parallel on wistar rats (n=4 per pseudotype). GFP expression was monitored by fluorescence fundus photography over a four-week period. Interestingly, different patterns of expression were obtained (Fig. 6A). HA-pseudotyped SIV showed the most efficient gene transfer in term of GFP expression level, which proved stable over at least 9 months when GFP was used as a transgene (Fig. 7). Vectors harboring MK-G, 4070A-Env and LCMV-G envelopes yielded lower transduction rates. HA, MK-G and 4070A-Env showed transgene expression kinetics similar to those obtained with the VSV-G with maximal GFP expression 6 days post-injection. In contrast, GFP signal increased slowly with time in LCMV-G SIV transduced retina. Finally, among the five vectors, RD114-Env pseudotype failed to transduce the retina.

To specify the cell populations actually transduced, two animals of each group were sacrificed at 14 days post injection and eyes were enucleated. Sclera/choroïd/RPE and neuroretina layers were analyzed under inverted fluorescence microscope. No signal could be detected with RD114, as expected. For all other SIV pseudotypes, the GFP fluorescence was restricted to the RPE cells (Fig. 6B), while no signal could be detected in the neuroretina.

Intravitreal injections (n=3 for each pseudotype) were also performed and monitored over a one month period, resulting in no detectable transduction (Fig. 8). Intravitreal injection of rAAV2.*gfp* was used as a positive control. SIV-injected animals were sacrificed 20 days post-injection. No GFP signal could be detected by fluorescence microscopy on retinal flatmounts and neuroretina cryosections. This result indicated that neuroretina cells such as ganglion or Müller cells exhibit a block during the infectious process for each of the pseudotypes tested.

### BIBLIOGRAPHY

Auricchio, A., G. Kobinger, et al. (2001). "Exchange of surface proteins impacts on viral vector cellular specificity and transduction characteristics: the retina as a model." Hum Mol Genet **10**(26): 3075-81.

Bainbridge, J. W., C. Stephens, et al. (2001). " In vivo gene transfer to the mouse eye using an HIV-based lentiviral vector; efficient long-term transduction of corneal endothelium and retinal pigment epithelium." Gene Ther **8**(21): 1665-8.

Desmaris, N., A. Bosch, et al. (2001). "Production and neurotropism of lentivirus vectors pseudotyped with lyssavirus envelope glycoproteins." Mol Ther **4**(2): 149-56.

Doi, K., J. Hargitai, et al. (2002). "Lentiviral transduction of green fluorescent protein in retinal epithelium: evidence of rejection." Vision Res **42**(4): 551-8.

Duisit, G., A. Salvetti, et al. (1999). "Functional characterization of adenoviral/retroviral chimeric vectors and their use for efficient screening of retroviral producer cell lines." Hum Gene Ther **10**(2): 189-200.

Hatziioannou, T., S. Valsesia-Wittmann, et al. (1998). "Incorporation of fowl plague virus hemagglutinin into murine leukemia virus particles and analysis of the infectivity of the pseudotyped retroviruses." J Virol **72**(6): 5313-7.

Mangeot, P. E., D. Negre, et al. (2000). "Development of minimal lentivirus vectors derived from simian immunodeficiency virus (SIVmac251 ) and their use for gene transfer into human dendritic cells." J Virol **74**(18): 8307-15.

Miyoshi, H., U. Blomer, et al. (1998). "Development of a self-inactivating lentivirus vector." J Virol **72**(10): 8150-7.

Miyoshi, H., M. Takahashi, et al. (1997). "Stable and efficient gene transfer into the retina using an HIV-based lentiviral vector." Proc Natl Acad Sci USA **94**(19): 10319-23.

Negre, D., P. E. Mangeot, et al. (2000). "Characterization of novel safe lentiviral vectors derived from simian immunodeficiency virus (SlVmac251) that efficiently transduce mature human dendritic cells." Gene Ther **7**(19): 1613-23.

Rolling, F., W. Y. Shen, et al. (1999). "Evaluation of adeno-associated virus-mediated gene transfer into the rat retina by clinical fluorescence photography." Hum Gene Ther **10**(4): 641-8.

Sandrin, V., B. Boson, et al. (2002). "Lentiviral vectors pseudotyped with a modified RD114 envelope glycoprotein show increased stability in sera and augmented transduction of primary lymphocytes and CD34+ cells derived from human and nonhuman primates." Blood **100**(3): 823-32.

## Claims

1. A lentiviral vector particle comprising a lentiviral vector genome encoding a transgene which, when expressed in the retina, can prevent or alleviate the effects of an eye disease, wherein said vector particle is pseudotyped with an hemagglutinin (HA) protein of an orthomyxovirus.

2. The lentiviral vector particle of claim 1, wherein said HA protein is from an influenza A virus.

3. The lentiviral vector particle of claim 2, wherein said HA protein is from a Fowl Plague Virus (FPV).

4. The lentiviral vector particle of claim 3, wherein said HA protein is from strain *influenza* A, FPV, Rostock, 34 (H7 N1), and has the sequence accessible in GenBank under number AAA43150.

5. The lentiviral particle of any of claims 1 to 4, further comprising a neuraminidase (NA) protein from an influenza virus inserted in its membrane.

6. The lentiviral particle of claim 5, wherein said NA protein is from an influenza A virus.

7. The lentiviral vector particle of claim 6, wherein said NA protein is from a Fowl Plague Virus (FPV).

8. The lentiviral vector particle of claim 7, wherein said HA protein is from strain *influenza A,* FPV, Rostock, 34 (H7 N1), and has the sequence accessible in GenBank under number CAA36475.

9. The lentiviral vector particle of any of claims 1 to 8, wherein said vector genome comprises a lentiviral 5' LTR, a tRNA binding site, a packaging signal, a promoter operably linked to said transgene, an origin of second strand DNA synthesis and a 3' lentiviral LTR.

10. The lentiviral vector particle of any of claims 1 to 9, wherein said vector genome comprises 5' and/or 3' LTRs from a virus selected from the group consisting of SIV, HIV, HIV-1, HIV-2, EIAV and FIV.

11. The lentiviral vector particle of any of claims 1 to 10, which is self-inactivating.

12. The lentiviral vector particle of any of claims 1 to 11, wherein said transgene is selected in the group consisting of neurotrophic factors including NGF, BDNF, CNTF, NT-3, NT-4, FGF-2, FGF-5, FGF-18, FGF-20 and FGF-21, anti-angiogenic factors including soluble Flt-1, soluble Tie-2 receptor, and PEDF, and enzymes including β-glucuronidase, neuraminidase, sphingomyelinase, sulfatases, arylsulfatase β, α-neuraminidase, gangliosidase, tripeptidyl protease, CLN3 and palmitoyl protein thioesterase (PPT).

13. The lentiviral vector particle of any of claims 1 to 12, wherein said transgene is operably linked to a regulated promoter, a photo-activated promoter, a viral promoter, a tissue specific promoter or a rhodopsin promoter.

14. A pharmaceutical composition for administration in the eye, comprising lentiviral vector particles according to any of claims 1 to 13, and a pharmaceutically acceptable carrier.

15. Use of a lentiviral vector particle according to any of claims 1 to 13, for the manufacture of a composition for treating a disease in the eye or preventing cell damage in the eye.

16. Use according to claim 15, wherein said disease is due to a deficiency in retinal pigment epithelium (RPE) cells.

17. Use according to claim 15, wherein said disease is due to a deficiency in the photoreceptors.

18. Use according to claim 17, wherein the lentiviral particles are able to transduce retinal pigment epithelium (RPE) cells and express a neurotrophic factor therein.

19. Use according to any of claims 15 to 18, for the manufacture of a composition for treating a neovascular disease of the eye, a glaucoma, a macular degeneration, a diabetic retinopathy, an inherited retinal degeneration, a retinitis pigmentosa, a cell damage in retinal epithelial cells associated with Sly syndrome, a retinal detachment or injury or a retinopathy.

20. Use of an hemagglutinin (HA) protein of an orthomyxovirus, for pseudotyping an enveloped vector so that said vector is able to transduce efficiently retinal pigment epithelium (RPE) cells.

21. Use according to claim 20, wherein said HA protein is from an influenza A virus.

22. Use according to claim 21, wherein said HA protein is from a Fowl Plague Virus.

23. Use according to claim 22, wherein the HA protein is from strain *influenza A,* FPV, Rostock, 34 (H7 N1), and has the sequence accessible in GenBank under number AAA43150.
